# EUROPEAN PATENT SPECIFICATION

(11) **EP 0 504 621 B1**
(45) Date of publication and mention of the grant of the patent: **11.12.1996**
(21) Application number: 92103043.3
(22) Date of filing: 24.02.1992
(51) Int. Cl.: C23C 8/12, C23C 8/14, C07C 273/04

(54) **Method for the passivation of metal surfaces affected by operating conditions and agents promoting corrosion**
Verfahren zum Passivieren von Metalloberflächen angegriffen durch Betriebsbedingungen und korrosionsförderende Medien
Méthode pour la passivation de surfaces de métaux attaquées par des conditions opérationelles et des agents favorisant la corrosion

(30) Priority: 18.03.1991 IT MI910715
(43) Date of publication of application: 23.09.1992
(73) Proprietor: UREA CASALE S.A., CH-6900 Lugano-Besso (CH)
(72) Inventor: Lagana, Vincenzo, I-20133 Milan (IT)
(74) Representative: Bottero, Claudio

(56) References cited:
- EP-A- 0 096 151
- EP-A- 0 096 151
- FR-A- 1 027 202
- wpil/derwent , abstract nr. 85-187612 c31, derwent publications,london,gb &jp-a-60116760 (HITACHI)24-06-85
- derwent publications,london,gb, nr.40982c/23 week c23 16-07-80 & su-a-446181(lengd elec eng inst) 15-10-79

## Description

This invention concerns a method for the passivation of metal surfaces in equipment affected by operating conditions and by agents which promote corrosion, in general of the surfaces in chemical plants involving the presence and formation of corrosive compounds especially where the environmental conditions (temperature and pressure) intensify said corrosive action.

More particularly the invention concerns a method for the passivation of the metal equipment used in chemical plants, and exposed to the intensive action of highly corrosive compounds, and what is more under temperature and pressure conditions higher than ambient conditions.

In an embodiment of great industrial interest, the invention consists in a method for the passivation of metal equipment used in urea synthesis and treatment processes.

Without affecting the generality of the invention, but only as a reference to one of the most interesting and immediate instances, in plants for the industrial production of urea through the synthesis of the reactants NH₃ and CO₂, with the formation of various compounds, besides urea, which are highly corrosive such as more or less concentrated aqueous solutions of ammonium carbamate, ammonium carbonate, urea itself and other kinds of ionic species.

The metal surfaces of the various parts of equipment which come into contact with the above-mentioned compounds are subjected to chemical aggression which affects their integrity and efficiency.

It is well known that the synthesis of urea is carried out at a high temperature (on average 180ö215°C) and high pressure (on average 130ö400 bar). Downstream the synthesis section are found a number of decomposition stages of urea synthesis by-products which have not been transformed into urea such as ammonium carbamate which through heat is decomposed into NH₃ and CO₂ and separated from the synthesis elements, as gaseous NH₃ and CO₂, which are condensed in successive condensation stages forming aqueous solutions of ammonium carbamate and/or ammonium carbonate which are recycled in the synthesis section, while the urea is concentrated in successive stages operating at decreasing pressure until the final vacuum concentration stage is reached from which is obtained virtually pure melted urea which is then sent to the finishing stage effected with various techniques.

Various systems have been put forward for the passivation of equipment used in the stages mentioned above which is subjected to aggression by the corrosive compounds treated in it. For example Belgian Patent No. 625.397 describes the use of oxygen as passivating agent at 180°C and 270 kg/cm² for the surfaces in a urea synthesis reactor in stainless steel containing up to 19% Cr and 14% Ni; in general oxygen is replaced by other passivating agents, for example hydrogen peroxide and alkaline metal peroxide or alkaline earth peroxide. In European Patent 0096151 a passivation system is described for strippers where the effluent from the urea synthesis reactor is treated at a high temperature and pressure, between 120 and 240 kg/cm², as a thin falling film countercurrent with NH₃ or CO₂; to a first passivating agent consisting of oxygen-containing gas introduced from the bottom of at least one stripper is added as second passivating agent a liquid injected from the top of the stripper and selected from hydrogen peroxide, alkaline metals persulphate or perborate, peracetic acid, organic peroxide.

Oxygen may be introduced into the plant as pure oxygen or mixed with air or with hydrogen peroxide.

The oxygen in gas form is introduced by injecting it into the CO₂ before this is compressed, or into the ammonia entering the synthesis zone, or as hydrogen peroxide into the various liquid flows upstream the entrance to the equipment to be protected.

The passivating system with hydrogen peroxide requires in any case the simultaneous injection of gaseous oxygen, either as air or as pure oxygen as mentioned before.

The above-mentioned passivation systems are used to protect from corrosion the metal material usually employed in industrial plants for the production of urea (various types of stainless steel, titanium, etc.).

According to FR 1.027.202, on the other hand, a process is described for obtaining a protective coating onto the metal surfaces totally or partially constituted by chromium, which comprises the step of heating the metal surface in an oxygen containing atmosphere, optionally enriched with ozone.

Besides the above-mentioned passivating agents others have been put forward (for example DE-A-1800755) such as soluble ammonium nitrite, soluble sodium nitrite, and other substances not used industrially.

Nowadays the technique universally adopted to passivate metal surfaces in contact with the solutions and vapours present in the plant's various stages is to send to the synthesis reactor air and oxygen by injecting them into the CO₂; in some cases, besides this injection, hydrogen peroxide is introduced into the liquid flow upstream of the equipment to be passivated.

The oxygen content injected into the CO₂ has a concentration of between 0.2% and 0.8% in volume and this causes some troubles of which just a few pointed out as follows:
1. the oxygen injected as air enriches the reactor with nitrogen, with the obvious consequence that the synthesis zone is enriched with inerts, thus creating a gas phase the consequence of which is a reduction in urea yield in the reactor;
2. since the CO₂ comes from the synthesis gas decarbonation section for the production of ammonia, it contains H₂, N₂, CO, CH₄ in such proportions that together with the oxygen they create explosive mixtures.

It has been seen that to escape the explosion field the amount of oxygen injected into CO₂ must be below 0.2% vol., but under these conditions it has also been observed that the protective action over the metal surfaces is greatly reduced thus leaving them exposed to the corrosive action of both liquid and gas substances present in the various parts of equipment.

The technical problem underlying the present invention is that of providing a method which eliminates the drawbacks of the present State of the Art and allows to overcome the situation of contrast between the necessity of having oxygen in large amount suitable for the desired passivation on one side and the necessity of avoiding the risks of explosion on the other side.

Surprisingly it has been found that to escape the field of explosion, the oxygen content may be reduced below 0.2% vol. without affecting the passivation of equipment, by means of adding to the passivation air small amounts of ozone (O₃).

Accordingly, the above-identified problem is solved by a method for the passivation of equipment used in urea synthesis and treatment, including urea synthesis reactors, concentration equipment, ammonium carbamate decomposition and condensation equipment, said equipment comprising stainless steel and titanium metal surfaces, which method comprises the step of dosing into the equipment metered amounts of a gaseous oxygen-containing passivating agent and is characterized in that said gaseous passivating agent has an oxygen content of from 0.01 to 0.2 % v/v on the volume of a carbon dioxide stream fed into said equipment and in that it further comprises the step of feeding into the equipment ozone as a second passivating agent in a quantity of from 0.01 to 0.1 % v/v on the volume of said carbon dioxide stream.

The synergic action of the oxydization of air and O₃ permits the achievement of, among others, the following advantages:
A. notable reduction of inerts in the urea synthesis reactor with the advantage of an increase in urea yield;
B. reduction of the vapour phase in the reactor thus increasing the volume of the liquid phase increasing its residence time;
C. avoidance of the field of explosion, since the O₂ does not create an explosive mixture with the H₂, CO, CH₄ present in the various stages of the plant.

The advantage of the invention is undeniable and significant all the more since the production of O₃ is achieved with amply experimented conventional methods, just as absolutely conventional is the system for eliminating O₃ from the gas inerts discharged into the atmosphere where the laws for the protection of the environment forbid its being released into the atmosphere, the production of O₃ and its elimination being a well-known technique.

The content of O₂ in the CO₂ may vary between 0.05% vol. and 0.2% vol. while the content of O₃ may vary between 0.01% vol. and 0.1% vol. When a compound selected from the group consisting of hydrogen peroxide (H₂O₂), alkaline metals peroxides, alkaline earth peroxides, alkaline metals persulphates or perborates, organic peroxides and acetic acid is present together with O₂ and O₃, the oxygen amount can be reduced below 0.05% vol., i.e. between 0.01% vol. and 0.05% vol. Preferably the third compound selected from the above group is hydrogen peroxide (H₂O₂).

A further advantage of the invention is that the concentration of O₃ may be maintained at a high level without the danger of explosions and with an appreciable increase in passivation, reducing the hydrogen (H₂) content (together with the CO, N₂ and CH₄ content) in the CO₂ obtained from the decarbonation plant of the ammonia synthesis gas, by preference through an additional pre-flash of the solution rich in CO₂ to be regenerated, before in effect it enters this regeneration stage.

The above pre-flash may be simply carried out at about 3 bar abs.

### EXAMPLES

The purpose of the following examples is to compare the composition of the CO₂ sent to the synthesis reactor and of the inert gas discharged into the athmosphere, both according to the known technique and to the invention.

We take into consideration in the case of a 1000 t/d urea plant the composition of the CO₂

### Comparison example 1.

### Known technique

The amount of CO₂ necessary for synthesis and its composition after the addition of air is as follows:

| | | |
|---|---|---|
| CO₂ | 15610 Nm³/h | 94.93 % vol. |
| N₂ | 491 Nm³/h | 2.98 % vol. |
| H₂ | 164 Nm³/h | 1.00 % vol. |
| O₂ | 98 Nm³/h | 0.60 % vol. |
| CO | traces Nm³/H | traces % vol. |
| CH₄ | 80 Nm³/h | 0.49 % vol. |
| Total | 16443 Nm³/h | 100.00 % vol. |

### Example 2. Invention

The amount of CO₂ necessary for synthesis and its composition after the addition of air enriched with O₃ is as follows:

| | | |
|---|---|---|
| CO₂ | 15610 Nm³/h | 96.77 % vol. |
| N₂ | 245 Nm³/h | 1.52 % vol. |
| H₂ | 164 Nm³/h | 1.02 % vol. |
| O₂ | 29 Nm³/h | 0.18 % vol. |
| O₃ | 3 Nm³/h | 0.02 % vol. |
| CO | traces Nm³/h | traces % vol. |
| CH₄ | 80 Nm³/h | 0.49 % vol. |
| Total | 16131 Nm³/h | 100.00 % vol. |

It can be seen that the amount of oxygen is appreciably reduced in the case of the invention as compared to the amount required for the known technique while the passivating effect is equally efficient thanks to the addition of ozone (O₃).

Composition of the inert gas discharged into the air in the two cases:

| | Known technique | | Invention | |
|---|---|---|---|---|
| N₂ | 491 Nm³h | 58.9 % vol. | 245 Nm³/h | 47.0 % vol. |
| H₂ | 164 Nm³/h | 19.7 % vol. | 164 Nm³/h | 31.5 % vol. |
| O₂ | 98 Nm³/h | 11.8 % vol. | 29 Nm³/h | 5.5 % vol. |
| O₃ | - | - | 3 Nm³/h | 0.6 % vol. |
| CO | traces | - | traces | - |
| CH₄ | 80 Nm³/h | 9.6 % vol. | 80 Nm³/h | 15.4 % vol. |
| | 833 Nm³/h | 100.0 % vol. | 521 Nm³/h | 100.0 % vol. |

The field of explosion of the two mixtures can easily be calculated by conventional method and it will be seen that the mixture according to the invention is outside the limits of explosion.

## Claims

1. A method for the passivation of equipment used in urea synthesis and treatment, including urea synthesis reactors, concentration equipment, ammonium carbamate decomposition and condensation equipment, said equipment comprising stainless steel and titanium metal surfaces, which method comprises the step of dosing into the equipment metered amounts of a gaseous oxygen-containing passivating agent, characterized in that said gaseous passivating agent has an oxygen content of from 0.01 to 0.2 % v/v on the volume of a carbon dioxide stream fed into said equipment and in that it further comprises the step of feeding into the equipment ozone as a second passivating agent in a quantity of from 0.01 to 0.1 % v/v on the volume of said carbon dioxide stream.

2. A method according to claim 1, characterized in that said gaseous passivating agent is air.

3. A method according to claim 1, characterized in that said gaseous passivating agent and said ozone are dosed in said carbon dioxide stream.

4. A method according to claim 1, characterized in that it further comprises the step of introducing into the equipment metered amounts of a compound selected from the group comprising hydrogen peroxide, alkaline metals peroxides, alkaline earths peroxides, alkaline metals persulphates or perborates, organic peroxides and acetic acid.

5. A method according to claim 4, characterized in that said gaseous passivating agent has an oxygen content of from 0.01 to 0.05 % v/v.

6. A method according to claim 1, characterized in that said gaseous passivating agent and said ozone are dosed into an ammonia or into a liquid flow fed into the equipment.

7. A method according to claim 6, characterized in that the ozone content in said ammonia or liquid flow varies from 2 to 1,000 ppm.

## Patentansprüche

1. Verfahren zum Passivieren von Einrichtungen, die bei der Harnstoffsynthese und -verarbeitung verwendet werden, einschließlich Harnstoffsynthesereaktoren, Konzentrationseinrichtungen, Ammoniumcarbamatzersetzungs- und Kondensationseinrichtungen, wobei die Einrichtung Oberflächen aus rostfreiem Stahl und Titanmetall aufweist, welches den Schritt des dosierten Eingebens von abgemessenen Mengen eines gasförmigen sauerstoffhaltigen Passivierungsreagenzes in die Einrichtung umfaßt, dadurch gekennzeichnet, daß das gasförmige Passivierungsreagenz einen Sauerstoffgehalt von 0,01% v/v bis 0,2% v/v, bezogen auf das Volumen eines Kohlendioxidstroms aufweist, welcher in die Einrichtung eingespeist wird, und daß es weiterhin den Schritt des Einspeisens von Ozon als zweites Passivierungsreagenz in die Einrichtung in einer Menge von 0,01% v/v bis 0,1% v/v, bezogen auf das Volumen des Kohlendioxidstroms, umfaßt.

2. Verfahren nach Anspruch 1, dadurch gekennzeichnet, daß das gasförmige Passivierungsreagenz Luft ist.

3. Verfahren nach Anspruch 1, dadurch gekennzeichnet, daß das gasförmige Passivierungsreagenz und das Ozon dosiert in den Kohlendioxidstrom eingegeben werden.

4. Verfahren nach Anspruch 1, dadurch gekennzeichnet, daß es weiterhin den Schritt des Einleitens von abgemessenen Mengen einer Verbindung in die Einrichtung umfaßt, die aus der Gruppe gewählt ist, welche Wasserstoffperoxid, Alkalimetall-Peroxide, Erdalkali-Peroxide, Alkalimetall-Persulphate oder -Perborate, organische Peroxide und Essigsäure umfaßt.

5. Verfahren nach Anspruch 4, dadurch gekennzeichnet, daß das gasförmige Passivierungsreagenz einen Sauerstoffgehalt von 0,01% v/v bis 0,05% v/v aufweist.

6. Verfahren nach Anspruch 1, dadurch gekennzeichnet, daß das gasförmige Passivierungsreagenz und das Ozon dosiert in Ammoniak oder in eine Flüssigkeitsströmung eingegeben werden, welche in die Einrichtung eingespeist werden.

7. Verfahren nach Anspruch 6, dadurch gekennzeichnet, daß der Ozongehalt in dem Ammoniak oder der Flüssigkeitsströmung von 2 ppm. bis 1000 ppm. variiert.

## Revendications

1. Méthode pour la passivation d'un équipement utilisé dans la synthèse et le traitement de l'urée, incluant des réacteurs de synthèse de l'urée, un équipement de concentration, un équipement de décomposition et de condensation de carbamate d'ammonium, ledit équipement comprenant des surfaces métalliques en acier inoxydable et en titane, cette méthode comprenant l'étape de dosage, à l'intérieur de l'équipement, de quantités mesurées d'un agent passivant contenant de l'oxygène gazeux, caractérisé en ce que ledit agent passivant gazeux a une teneur en oxygène de 0,01 à 0,2 % v/v sur le volume du courant de dioxyde de carbone conduit à l'intérieur dudit équipement et en ce qu'il comprend en outre l'étape de conduite d'ozone à l'intérieur de l'équipement comme second agent passivant, selon une quantité de 0,01 à 0,1 % v/v sur le volume dudit courant de dioxyde de carbone.

2. Méthode selon la revendication 1, caractérisée en ce que ledit agent passivant gazeux est l'air.

3. Méthode selon la revendication 1, caractérisée en ce que ledit agent passivant gazeux et ledit ozone sont dosés dans ledit courant de dioxyde de carbone.

4. Méthode selon la revendication 1, caractérisée en ce qu'elle comprend en outre l'étape d'introduction à l'intérieur de l'équipement de quantités mesurées d'un composé choisi parmi le groupe comprenant le peroxyde d'hydrogène, les peroxydes de métaux alcalins, les peroxydes d'alcalino-terreux, les persulfates ou perborates de métaux alcalins, les peroxydes organiques et l'acide acétique.

5. Méthode selon la revendication 4, caractérisée en ce que ledit agent passivant gazeux a une teneur en oxygène de 0,01 à 0,05 % v/v.

6. Méthode selon la revendication 1, caractérisée en ce que ledit agent passivant gazeux et ledit ozone sont dosés dans un flux d'ammoniaque ou de liquide conduit à l'intérieur de l'équipement.

7. Méthode selon la revendication 6, caractérisée en ce que la teneur en ozone dans ledit flux d'ammoniaque ou de liquide varie de 2 à 1000 ppm.
